# EUROPEAN PATENT SPECIFICATION

(11) **EP 2 234 611 B1**
(45) Date of publication and mention of the grant of the patent: **08.02.2012**
(21) Application number: 08868549.0
(22) Date of filing: 17.12.2008
(51) Int. Cl.: A61K 31/197, A61K 31/198, A61K 31/519, A61K 33/04, A61K 33/24, A61K 33/30, A61K 35/30, A61K 45/06

(54) **COMPOSITION FOR TREATING STERILE INFLAMMATION**
ZUSAMMENSETZUNG ZUR BEHANDLUNG STERILER ENTZÜNDUNGEN
COMPOSITION POUR LE TRAITEMENT DE L'INFLAMMATION STÉRILE

(30) Priority: 28.12.2007 FI 20075971
(43) Date of publication of application: 06.10.2010
(73) Proprietor: Oy Neurofood Ab, 02270 Espoo (FI)
(72) Inventor: TALLBERG, Thomas, FI-00200 Helsingfors (FI)
(74) Representative: Boije-Backman, Solveig Magdalena
(86) International application number: PCT/FI2008/050750
(87) International publication number: WO 2009/083643

(56) References cited:
- EP-A- 0 737 471
- EP-A- 1 523 985
- WO-A-2005/074910
- TALLBERG T ET AL: "Development of a Combined Biological and Immunological Cancer Therapy Modality" JOURNAL OF THE AUSTRALASIAN COLLEGE OF NUTRITIONAL ANDENVIRONMENTAL MEDICINE, COLLEGE, BEAUMARIS, AU, vol. 22, no. 1, 1 April 2003 (2003-04-01), pages 3-21, XP002987380 ISSN: 1328-8040

## Description

### Field of the Invention

The present invention relates to a use of natural biological components for the manufacture of a pharmaceutical composition for treating or preventing sterile inflammation. More specifically the invention relates to a use of L-amino acids arginine (Arg) or lysine (Lys) and leucine (Leu), trace elements Cr, Sn, Se, Sr, V, and W, and folic acid for treating or preventing sterile inflammation.

### Background of the Invention

Disturbances in the normal lymphopoiesis results in several sterile inflammatory diseases. These frequently manifesting ailments appear in many varieties, characterized by severe pathologic tissue reactions affecting internal organs often manifested as chronic illnesses. These forms of deleterious inflammatory reactions, some with increasing incidence, are listed according to their various diagnoses as: 1] fibromyalgia, M79.0/M79.9 (classification according to WHO, ICD-10). Yearly incidence in 2006 in Finland 2273 cases. 2] Crohn's disease K50.1 / K50.9., Yearly incidence 2110 cases 3] rheuma M06.0 / M06.9 / M05.9 / M07.0. Incidence 1454 cases / year. 4] psoriasis L40 / L40.5 L40.9., Incidence 930 / year. These diseases seem to be linked to a certain genetic susceptibility and stress. The treatment of these patients is usually complicated and expensive, although curative results are not regularly achieved. In Crohns' disease e.g. the intestinal tract is involved, and when the colon is affected this emerges as colitis ulcerosa. When certain of these diseases become progressive, they may also affect the joints and cause severe pain, restricting the patients' mobility. Standard therapies have not lately improved appreciably, a usual remedy still being light therapy, certain ointments, cortisone, different anti-inflammatory medicines and, ultimately, even cytostatics. No truly effective natural medication has been devised. There is a great demand for improved methods for treating these diatheses with methods that are effective, simple, inexpensive, and without harmful side effects.

As these chronic inflammations sometimes require great resources, such resources have also been spent on finding remedies for this disease group. Conventionally these diseases are treated by surgery, with anti-inflammatory agents or cytostatics to prevent malignant transformation. Alternative methods of treatment include (for example) a combined biological and immunological treatment modality called bio-immunotherapy (Tallberg, Thomas. Development of a Combined Biological and Immunological Cancer Therapy Modality. A review of Bio-Immunotherapy. J. Austr. Coll. Nutr. & Env. Med. 2003:22 p. 3 to 21. / Regulation of cancer by therapeutic vaccination and dietary bio-modulation involving organ specific mitochondria. In, Int. J. Biotechnology Vol.9, Nos. 3/4, 391-409. 2007). In bio-immunotherapy, these patients are given natural biological components which consist of a mixture of amino acids and trace elements, optionally in combination with neurogenic lipids and/or vitamins. Varying mixtures of these ingredients have been tested for different forms of these diatheses. The mixtures are preferably given as a functional food supplement.

### Brief Description of the Invention

The invention relates to a pharmaceutical composition comprising neurogenic CNS lipids L-amino acids arginine (Arg) or lysine (Lys) and leucine (Leu), combined with trace elements chromium (Cr), tin (Sn), selenium (Se), strontium (Sr), vanadium (V), and wolfram (W), and with folic acid as pharmaceutically active ingredients for treatment and/or prevention of sterile inflammation, in relation to aliments selected from the group consisting of fibromyalgia, Crohn's disease and rheuma.

The invention further relates to a pharmaceutical composition comrpising as sole pharmaceutically active ingredients:
a) L-amino acids arginine (Arg) or lysine (Lys) and leucine (Leu),
b) trace elements chromium (Cr), tin (Sn), selenium (Se), strontium (Sr), vanadium (V), wolfram (W), and manganese (Mn),
c) folic acid,
d) CNS -lipids.

Some preferred embodiments of the invention are set forth in the dependent claims.

The present invention provides an inexpensive and safe pharmaceutical composition. The invention provides an alternative composition for treating or compensating the causative metabolic deficiency forming the etiology of sterile inflammation by administering natural, biological components to a subject in need thereof.

### Detailed Description of the Invention

The present invention is based on the unexpected finding that during the treatment of cancer patients with bio-immunotherapy, it became apparent in several patients that their disparate inflammatory symptoms were appreciably mitigated. The positive effect seemed to be independent of the malignant disease.

The present invention provides a use of natural, biological components for the manufacture of a pharmaceutical composition for treating or preventing sterile inflammation. The pharmaceutical composition is especially useful for treating and/or preventing sterile inflammation in relation to disorders such as fibromyalgia, Crohn's disease, rheuma.

The invention relates to a pharmaceutical composition comprising natural biological components, optionally including central nervous system (CNS) lipid factors, as pharmaceutically active ingredients, involved in amending normal lymphopoiesis and also thereby responsible for regulating inflammatory reactions in various tissues. This composition has a curing and prophylactic effect on these inflammatory diseases of unknown aetiology. It is aimed to correct the complex metabolic deficiency causing this aberration of the immune system.

The present invention thus relates to the use of
a) L-amino acids arginine (Arg) or lysine (Lys) and leucine (Leu),
b) trace elements chromium (Cr), tin (Sn), selenium (Se), strontium (Sr), vanadium (V), and wolfram (W),
c) folic acid,
d) neurogenic CNS lipids
for the manufacture of a pharmaceutical composition for treating or preventing sterile inflammation. The pharmaceutical composition may prefereably further comprise zinc (Zn), manganese (Mn) and/or neurogenic CNS lipids.

When analysing patients who suffered from these ailments and the method biological components the patients had ingested, it became evident that certain amino acids together with specific essential trace element metal ions used in the bio-immunotherapy they received were responsible for a positive clinical effect on their disease, i.e. diminished symptoms. In close family members of these cancer patients, who were otherwise healthy but who also had suffered from bouts of fibromyalgia, rheumatoid arthritis, or Crohns' disease, the same natural dietary components ingested could cause a favorable clinical effect.

Therefore patients who were not suffering from cancer were therefore studied. In numerous cases tested the active dietary components have been delineated. An oral intake prescribed, in the form of a food supplementation to these patients and it is based on a formulation comprising a combination of the amino acids leucine (Leu) and arginine (Arg) or lysine (Lys). Said two amino acids are administered in the form of L-amino acids.

In addition to Leu and Arg (as hydrochloride for the sake of taste) the composition preferably also comprises at least one of the essential trace elements selected from the group consisting of chromium (Cr), tin (Sn), selenium (Se), strontium (Sr), vanadium (V), wolfram (W). Optionally the trace elements also include e.g. manganese (Mn) and zinc (Zn). To be biologically active the trace elements should be in ionic form, wherefore in practice they are administered as salts. A salt having a neutral taste is preferred, and salts having a strong or bad taste should be avoided.

The formulation for treating or preventing said inflammatory syndromes further comprises physiologic amounts of folic acid.

The treatment as described herein successfully used is based on oral administration of L-arginine-hydrochloride, and L-leucine, optionally together with essential trace elements and folic acid as active ingredients. According to one preferred embodiment of the invention, the composition consists essentially of four groups of components, i.e. amino acids, trace elements, vitamins and central nervous system (CNS) lipids. All these components are naturally occurring and biologically active, which means that they participate in biological events such as metabolic processes. Very good clinical results were obtained with a composition comprising L-arginine, L-leucine, salts of Cr, Se, Sn, Sr, V, W, Mn, and folic acid as sole pharmaceutically active ingredients.

For a better result, in certain cases the amino acid arginine was exchanged for another basic amino acid, namely lysine.

The composition used comprises the components in biologically and pharmaceutically active amounts, that is amounts sufficient to achieve the desired health promoting effect. As will be readily understood by a physician, the amounts will vary depending on the individual and his or her health status as well as on other factors such as weight, age, nutrition, stress, environmental factors, etc. Examples of suitable amounts include, but are not limited to, about 2 to 10, usually 2 to 5, g/day of each of L-arginine-hydrochloride and L-leucine. The trace elements are usually used in amounts of 0.5 to 9 mg/day, preferably 1 to 3 mg/day of each of the trace element ions of Cr, Se, Sn, Sr, V and W. Manganese (Mn) may be administered in doses of about 50 mg/day. Folic acid is used in small, well-established physiological amounts, such as 1 to 2 mg/day.

Symptoms of mental depression are common in patients suffering from sterile inflammation and especially the inflammatory disorders listed above. CNS -lipids have been shown to aleviate symptoms of mental depression. Prion-free neurogenic CNS -lipids can also be administered to all patients suffering from the above-listed inflammatory disorders for prevention and treatment of mental depression. Patients suffering from mental depression can also be treated with the above-disclosed composition. These vital lipids are obtained from an animal that does not develop "mad cow disease". Therefore, CNS -lipids are obtained e.g. from the brain of healthy young pigs. The brain tissue is boiled, frozen and lyophilised, whereafter the lipids are extracted with ether-ethyl alcohol as previously described to obtain a prion-free lipid fraction (Tallberg T. et al. Cancer Immunity. The Effect in Cancer-Immunotherapy of Polymerised Autologous Tumour Tissue and Supportive Measures. Scand. J. Lab. Invets. 1979:39; 3 to 33). Neurogenic lipids are purchased and canned by Neurofood Ltd., Finland. The recommended daily intake of CNS-lipids is equivalent to about 50 to 100 g brain until the inflammatory symptoms have subsided.

The ingredients in the composition of this functional food-item including amino acids, trace elements, vitamins, and CNS -lipids can be administered as such either separately or in varying combinations. They may be purchased e.g. in powder form separately, or as ready made powders containing all ingredients. Such a powder mixture may be pre-packed and used as such or as a supplement to conventional food items e.g. in the patient's morning yoghurt. For the consumer it is easy to ingest in connection with breakfast or as a snack between meals. This compensatory dietary treatment is comparatively inexpensive, since it consists of natural components only, forming the active food additive. Of course the pharmaceutical composition may also be processed into granulates, capsules or tablets, which may comprise pharmaceutically acceptable carriers. The neurogenic lipid product can be administered either simultaneously with the other ingredients or separately at a different time. A preferred way is to mix the neurogenic lipid product with assorted fruits and ingest it chilled or as ice-cream.

Another aspect of the present invention is to provide a pharmaceutical composition comprising as sole pharmaceutically active ingredients:
a) L-amino acids arginine (Arg) or lysine (Lys) and leucine (Leu),
b) trace elements chromium (Cr), tin (Sn), selenium (Se), strontium (Sr), vanadium (V), and wolfram (W), and manganese (Mn),
c) folic acid,
d) neurogenic CNSlipids.
The pharmaceutical compositin may further comprise the amino acids serine (Ser) and isoleucine (Ile).
A composition for use in treating or preventing sterile inflammation by administering to a patient In need thereof a pharmaceutically active amount of
a) L-amino acids arginine (Arg) or lysine (Lys) and leucine (Leu),
b) trace elements chromium (Cr), tin (Sn), selenium (Se), strontium (Sr), vanadium (V) wolfram (W), and optionally zinc (Zn) and or manganese (Mn), and
c) folic acid, and optionally
d) CNS-lipids,
is also disposed.

The invention is illustrated by the following example.

### Example

Patients (who were) suffering from the above-listed sterile inflammatory ailments were given orally, in the form of a food supplementation, leucine and arginine together with milligram amounts of certain biologically active trace -element salts, in which the metal ions are present in a biologically active form, and including 50 to 100 g/d of prion-free CNSlipids. Some milligrams of folic acid were also included in the composition. This natural food supplementation was given to the patients every day. The dietary bio-modulation schedule for treatment of patients suffering from these sterile inflammations or mental depression was as follows:

### Combined supportive dietary measures:

1. Oral administration of (2 to 5 g/day) of each of the respective L-amino acids; arginine (Arg), and leucine (Leu) in connection with meals.
2. Essential trace element salts orally as biologically active ions, at dose levels of some milligrams (1 to 3 mg/day); chromium (CrCl₃ · 6 H₂0) 6mg (= 1.17 mg Cr), tin (SnCl₄ · 5 N₂O) 4mg (= 1.35mg Sn), selenium Na₂SeO₄ · 10 H₂O, 6mg (= 1.28 mg Se), vanadium (Na₂VO₄. 4 H₂O), 6mg (= 2.5 mg V), wolfram (Na₂WO₄ · 2 H₂O), 4mg (= 2.3mg W), strontium (SrCl₂) (=2mg Sr). Some patients also received in daily amounts of 140 mg/day of manganese sulphate corresponding to (= 51.0 mg Mn).
3. Small physiologic amounts of folic acid (1 to 2 mg/day).
4. The patients received a diet additionally containing prion-free CNS neurogenic lipids equivalent to approx. 50 g of brain (purchased and canned by Neurofood Ltd., Finland) mixed with fruits, or as ice-cream for the sake of taste.
5. The patients received pre-packed powders comprising a mixture of ingredients 1 to 3, and were recommended to mix the powder into their morning yoghurt to get their daily ration. The CNS -lipids in lyophilized form can be added or ingested separately.
6. Dose -levels were adjusted based on a clinical response observed, and correlated to the patients' body weight.

In numerous patients tested, the positive clinical effect of the treatment in numerous patients tested has lasted for over 25 years under continuous or intermittent administration of this bio-modulating composition. The effect was registered in some months time. Pain and bleeding could subside and laboratory values normalize. Joint pain subsided. The positive clinical outcome showed slight individual differences pertaining to the quantity and relative content of these biological ingredients, and the patients' body weight. In certain cases, Leu and Arg combined with CNS -lipids alone could cause a certain positive effect, but in other patients trace -element ions seemed to be necessary co-factors.

This variation may be linked to certain genetic traits in patients suffering from these inflammatory diatheses. This dietary treatment, aimed to compensate a metabolic deficiency underlying these diseases, was not linked to any side effects experienced in any of the volunteers tested. This food additive can exert a lasting mitigating effect on patients suffering from these ailments.

## Claims

1. Pharmaceutical composition comprising
a) neurogenic CNS lipids
b) L-amino acids arginine (Arg) or lysine (Lys) and leucine (Leu),
c) trace elements chromium (Cr), tin (Sn), selenium (Se), strontium (Sr), vanadium (V), and wolfram (W), and
d) folic acid
for use in treating or preventing sterile inflammation, in relation to ailments selected from the group consisting of fibromyalgia, Crohn's disease, and rheuma.

2. The pharmaceutical composition for use according to claim 1, **characterized in that** it further comprises zinc (Zn).

3. The pharmaceutical composition according to claim 1 or 2, **characterized in that** it further comprises manganese (Mn).

4. The pharmaceutical composition according to any one of claims 1 to 3, **characterized in that** it comprises:
a) neurogenic CNS lipids
b) 2 to 5 g of each of L-arginine-hydrochloride and L-leucine,
c) 0.5 to 9 mg of each of chromium (Cr), tin (Sn), selenium (Se), strontium (Sr), vanadium (V), and wolfram (W) salts, and
d) 1 to 2 mg of folic acid.

5. The pharmaceutical composition according to any one of claims 1 to 4, **characterized in that** it is in powder form.

6. The pharmaceutical composition according to any one of claims 1 to 5, **characterized in that** it is in the form of a functional dietary supplementation composition.

7. The pharmaceutical composition according to claim 1, **characterized in that** it further comprises serine (Ser) and isoleucine (Ile).

8. A pharmaceutical composition, **characterized in that** it comprises as pharmaceutically active ingredients:
a) neurogenic CNS -lipids
b) L-amino acids arginine (Arg) or lysine (Lys) and leucine (Leu),
c) trace elements chromium (Cr), tin (Sn), selenium (Se), strontium (Sr), vanadium (V), and wolfram (W), zinc (Zn), and manganese (Mn), and
d) folic acid.

9. The pharmaceutical composition according to claim 8, **characterized in that** it further comprises serine (Ser) and isoleucine (Ile) as pharmaceutically active ingredients.

## Patentansprüche

1. Pharmazeutische Zusammensetzung, umfassend:
a) neurogene ZNS-Lipide
b)L-Aminosäuren: Arginin (Arg) oder Lysin (Lys) und Leucin (Leu),
c)Spurenelemente: Chrom (Cr), Zinn (Sn), Selen (Se), Strontium (Sr), Vanadium (V) und Wolfram (W) und
d)Folsäure
zur Verwendung bei der Behandlung und der Vorbeugung aseptischer Entzündung in Verbindung mit Erkrankungen, ausgewählt aus der Gruppe, bestehend aus Fibromyalgie, Morbus Crohn und Rheuma.

2. Pharmazeutische Zusammensetzung zur Verwendung gemäß Anspruch 1, **dadurch gekennzeichnet, dass** sie des Weiteren Zink (Zn) umfasst.

3. Pharmazeutische Zusammensetzung gemäß den Ansprüchen 1 oder 2, **dadurch gekennzeichnet, dass** sie des Weiteren Mangan (Mn) umfasst.

4. Pharmazeutische Zusammensetzung gemäß einem jeden der Ansprüche 1 bis 3, **dadurch gekennzeichnet, dass** sie umfasst:
a) neurogene ZNS-Lipide
b)2 bis 5 g jeweils von L-Argininhydrochlorid und L-Leucin,
c) 0, 5 bis 9 mg jeweils von Chrom (Cr)-, Zinn (Sn)-, Selen (Se)-, Strontium (Sr)-, Vanadium (V)- und Wolfram (W)-Salzen und
d)1 bis 2 mg Folsäure.

5. Pharmazeutische Zusammensetzung gemäß einem jeden der Ansprüche 1 bis 4, **dadurch gekennzeichnet, dass** sie in Pulverform vorliegt.

6. Pharmazeutische Zusammensetzung gemäß einem jeden der Ansprüche 1 bis 5, **dadurch gekennzeichnet, dass** sie in Form einer funktionalen Nahrungsergänzungsmittelzusammensetzung vorliegt.

7. Pharmazeutische Zusammensetzung gemäß Anspruch 1, **dadurch gekennzeichnet, dass** sie des weiteren Serin (Ser) und Isoleucin (Ile) umfasst.

8. Pharmazeutische Zusammensetzung **dadurch gekennzeichnet, dass** sie als pharmazeutische Wirkstoffe
a) neurogene ZNS-Lipide
b)L-Aminosäuren: Arginin (Arg) oder Lysin (Lys) und Leucin (Leu)
c)Spurenelemente: Chrom (Cr), Zinn (Sn), Selen (Se), Strontium (Sr), Vanadium (V) und Wolfram (W), Zink (Zn) und Mangan (Mn) und
d)Folsäure umfasst.

9. Pharmazeutische Zusammensetzung gemäß Anspruch 8, **dadurch gekennzeichnet, dass** sie des Weiteren Serin (Ser) und Isoleucin (Ile) als pharmazeutische Wirkstoffe umfasst.

## Revendications

1. Composition pharmaceutique comprenant
a) des lipides CNS neurogènes
b) des L-amino acides arginine (Arg) ou lysine (Lys) et leucine (Leu),
c) des éléments trace de chrome (Cr), étain (Sn), sélénium (Se), strontium (Sr), vanadium (V), et tungstène (W), et
d) de l'acide folique
pour une utilisation dans le traitement ou la prévention de l'inflammation stérile, en relation avec des affections appartenant au groupe constitué de la fibromyalgie, de la maladie de Crohn, et du rhumatisme.

2. La composition pharmaceutique pour une utilisation selon la revendication 1, **caractérisée en ce qu'**elle comprend en outre du zinc (Zn).

3. La composition pharmaceutique selon les revendications 1 ou 2, **caractérisée en ce qu'**elle comprend en outre du manganèse (Mn).

4. La composition pharmaceutique selon l'une quelconque des revendications 1 à 3, **caractérisée en ce qu'**elle comporte:
a) des lipides CNS neurogènes
b) de 2 à 5 g de chacun des L-arginine chlorhydrate et L-leucine,
c) de 0,5 à 9 mg de chacun des sels de chrome (Cr), étain (Sn), sélénium (Se), strontium (Sr), vanadium (V), et tungstène (W), et
d) de 1 à 2 mg d'acide folique.

5. La composition pharmaceutique selon l'une quelconque des revendications 1 à 4, **caractérisée en ce qu'**elle est sous forme de poudre.

6. La composition pharmaceutique selon l'une quelconque des revendications 1 à 5, **caractérisée en ce qu'**elle est sous la forme d'une composition fonctionnelle de suppléments alimentaires.

7. La composition pharmaceutique selon la revendication 1, **caractérisée en ce qu'**elle comprend en outre de la sérine (Ser) et de l'isoleucine (Ile).

8. Une composition pharmaceutique, **caractérisée en ce qu'**elle comprend des ingrédients pharmaceutiquement actifs:
a) des lipides CNS neurogènes
b) des L-aminoacides arginine (Arg) ou lysine (Lys) et leucine (Leu),
c) des éléments trace de chrome (Cr), étain (Sn), sélénium (Se), strontium (Sr), vanadium (V), tungstène (W), zinc (Zn), et manganèse (Mn), et
d) de l'acide folique.

9. La composition pharmaceutique selon la revendication 8, **caractérisée en ce qu'**elle comprend en outre de la sérine (Ser) et de l'isoleucine (Ile) en tant qu'ingrédients pharmaceutiquement actifs.
